# EUROPÄISCHE PATENTSCHRIFT

(11) **EP 2 205 176 B1**
(45) Veröffentlichungstag und Bekanntmachung des Hinweises auf die Patenterteilung: **07.09.2011**
(21) Anmeldenummer: 08804081.1
(22) Anmeldetag: 12.09.2008
(51) Int. Cl.: A61C 1/00, A61C 19/04, A61B 19/00

(54) **VERFAHREN ZUR POSITIONSBESTIMMUNG EINES INTRAORAL MESSENDEN MESSGERÄTES**
METHOD FOR DETERMINING THE POSITION OF AN INTRAORAL MEASURING DEVICE
PROCÉDÉ DE DÉTERMINATION DE LA POSITION D'UN APPAREIL DE MESURE DESTINÉ À DES MESURES INTRAORALES

(30) Priorität: 12.09.2007 DE 102007043366
(43) Veröffentlichungstag der Anmeldung: 14.07.2010
(73) Patentinhaber: DeguDent GmbH, 63457 Hanau (DE)
(72) Erfinder: ERTL, Thomas, 63303 Dreieich (DE)
(74) Vertreter: Stoffregen, Hans-Herbert
(86) Internationale Anmeldenummer: PCT/EP2008/062116
(87) Internationale Veröffentlichungsnummer: WO 2009/034157

(56) Entgegenhaltungen:
- EP-A- 0 741 994
- EP-A- 0 951 874
- WO-A-02/083257
- US-A- 4 673 352
- US-A- 5 897 509
- US-B1- 6 381 485

## Beschreibung

Die Erfindung bezieht sich auf ein Verfahren zur Positionsbestimmung eines relativ zum Kauorgan eines Patienten zu bewegenden intraoral messenden Messgerätes, mit dem Positionen im Kauorgan oder Bereiche des Kauorgan gemessen werden, wobei die Position des Messgerätes mittels eines in ortsfester Beziehung zu dem Messgerät stehenden Positionsbestimmungssensors gemessen wird. Auch bezieht sich die Erfindung auf Verfahren und Anordnungen zum Bewerten wie Messen der Position und Taschentiefe einer parodontalen Zahnfleischtasche.

Aus der EP-A-1733 693 (Goldbach) ist ein medizinisches Tracking System bekannt, das auf Infrarot Basis arbeitet. Es benötigt jedoch wie andere Systeme (z. B. EP-A-1 523 950 (Foley)) einen zusätzlichen Ständer mit Infrarot Sendern und Empfängern im Operationsraum zur Referenzierung. Diese Systeme verknüpfen Daten aus bildgebenden Verfahren und die Positionsinformation des Navigationssystems zur Positionierung von Instrumenten für stereotaktische Operationen z. B. in der Neurochirurgie.

In der Zahnheilkunde berichten erste Veröffentlichungen über Anwendung oben genannter Systeme zur Implantatpositionierung (Mischkowski et al.: Comparison of static and dynamic computer-assisted guidance methods in implantology, in Int. J. Comput. Dent. 2006 Jan; 9(1):23-35). Auch hier werden Bilddaten mit Navigationsdaten verknüpft, um die Bohrrichtung für das Implantat zu bestimmen.

Marmulla R. et al: Intraoperative precision of mechanical, electromagnetic, infrared and laser-guided navigation systems in computer-assisted surgery, in Mund Kiefer Gesichtschir. 1998 May; 2 Suppl 1: S145-8, beschreibt die Anwendung eines Navigationssystems auf elektromagnetischer Basis. Dabei werden die unpräzisen Resultate in Gegenwart metallischer Gegenstände bemängelt.

Beim intraoralen Scannen von Zähnen ist der sichtbare Teilbereich eines Zahns oder Kieferabschnitts, von dem die 3D-Daten gemessen werden, zumeist viel kleiner als der gesamte Zahn oder Kiefer, so dass die Notwendigkeit entsteht, mehrere Bilder aus verschiedenen Blickrichtungen zu einem Gesamtdatensatz des Zahns oder Kieferabschnitts zu vereinigen.

Die Schwierigkeit der genauen Ortsbestimmung der Bereiche des Kauorgans, also Unter- und Oberkiefer, der Zähne sowie sonstiger interessierender Bereiche wie Taschen liegt zudem in dem Umstand begründet, dass sich nicht nur Ober- und Unterkiefer zueinander bewegen, sondern der Patientenkopf selbst und zusätzlich der von Hand geführte Scanner zum Kiefer selbst bewegt wird. Dabei erfolgen Aufnahmen von Kieferbereichen in unterschiedlichen Stellungen und Winkeln, so dass sich ungeachtet ausgereifter Software zum Mergen Fehler ergeben können.

Nach dem Stand der Technik wird das softwaremäßig Zusammenfügen bzw. -setzen der Einzelaufnahmen auch dadurch erschwert, dass die Zähne keine klaren Kanten und Ecken aufweisen. Wenn man zudem die Positionsbeziehung der Aufnahmen zueinander (die Kameraposition) nicht kennt, entsteht ein erheblicher Rechenaufwand, der z. B. bei dem Intraoral Scanning System der Firma CaDent deutlich länger als 30 Minuten beträgt, da die Algorithmen bei völlig unbekannter Kameraposition und der nicht kooperativen Zahngeometrien nur langsam konvergieren.

Dies führt zu inakzeptablen Wartezeiten für Behandler und Patient bei der Beurteilung des elektronischen Abdrucks und der Entscheidung, ob der Vorgang ggf. wiederholt werden muss.

Um dieses Problem zu lösen, können Referenzobjekte in den Mund eingebracht werden (US-B-7,065,243). Dabei wird durch intraorales Messen eines oder mehrerer Zähne bei gleichzeitiger Gegenwart einer Referenz durch diese die Position einer Kamera bestimmt, mittels der der Zahn bzw. die Zähne gemessen werden. Nach Kenntnis der Kameraposition in Bezug auf den Zahn bzw. die Zähne wird sodann aus den Messungen ein 3D-Modell erzeugt, das für die Herstellung von Zahnersatz benötigt wird. Ein entsprechendes Verfahren ist aufwändig und zeigt zudem den Nachteil, dass die als quaderförmiger offener Kasten ausgebildete Referenz den Zahn bzw. die Zähne abschattet.

Auch die US-A-2003/0219148 bezieht sich auf ein Verfahren zur Generierung eines dreidimensionalen Modells eines Zahnbogens unter Zuhilfenahme dreidimensionaler Referenzierungen.

Aus der US-A-2006/0212260 ist ein Verfahren bekannt, um intraoral zu scannen. Hierzu wird ein Scanner benutzt, der ein Inertial- oder Trackingsystem umfasst, um Lage und Position zu bestimmen.

Die US-A-2005/0020910 sieht bei einem intraoralen Messsystem die Möglichkeit vor, bei dem sowohl ein Scanner als auch ein am Kopf eines Nutzers befestigter Empfänger mit einem dreidimensionalen Trackingsystem versehen wird.

Die DE-B-100 45 381 bezieht sich auf eine Vorrichtung zur Bestimmung der Position eines medizinischen Instruments oder Gerätes oder Körperteils. Hierzu weist der Gegenstand, dessen Position bestimmt werden soll, aktive und passive Referenzkörper auf, um mit Navigationssystemen eine Positionsbestimmung vorzunehmen. Dabei können Neigungs- und Magnetfeldsensoren eingesetzt werden.

Aus der EP-A-1 088 525 sind ein Verfahren sowie eine Vorrichtung zur Justierung medizinischer Bilder auf einem Patienten zu entnehmen. Hierzu sind mit dem Patientenkörper verbindbare Halteelemente vorgesehen, die mit Markier- bzw. Lokalisiermitteln versehen sind. Hierdurch soll für die computergestützte Chirurgie ein dreidimensionales Lokalisierungssystem geschaffen werden, um die Ausrichtung zu medizinischchirurgischen Handlungen zu ermöglichen.

Bei der Messung parodontaler Taschentiefen oder bei der Kariesdiagnostik wird dem Stand der Technik entsprechend keine Augmentation mit Positionsdaten genutzt. Damit sind keine automatischen Verfahren zur Überführung der Messdaten in das Patientenverwaltungsprogramm der Praxis möglich.

Zur Feststellung von Parodontalerkrankungen erfolgt heutzutage ein Arbeiten dahingehend, dass in die Zahnfleischtaschen manuell eine Messsonde eingeführt und die Tiefe mittels einer Skala der Sonde durch Ablesen bestimmt wird. Vom Ort der Messung wird sodann vom Arzt einer Assistentin der Wert mitgeteilt, die die entsprechenden Daten in Abhängigkeit von dem Zahn bzw. Position der Messung zu einem Zahn notiert. Dies ist zeit- und personalaufwändig.

Bei der Kariesdiagnostik wird ebenso die Diagnose mit Angabe der Lokalität am Zahn verbal übermittelt oder vom Behandler selbst manuell in den Praxiscomputer eingegeben. Neben Zeitverlust ist dies zudem hygienisch bedenklich, da parallel zur Arbeit am Patienten eine Tastatur zu bedienen ist, die oft nicht hinreichend keimfrei zu halten ist.

Dem Stand der Technik nach ist lediglich ein elektromechanisches Gerät zur Erfassung der Taschentiefe bekannt, das aber keine Positionsdaten ermittelt. (Florida probe www.floridaprobe.de). Die Taschentiefe wird durch einen mechanischen Anschlag ermittelt, durch den die stiftförmige Sonde gleitet, bis sie den Taschenboden berührt. Die Wegstrecke, die der Stift bis zum Taschenfundus benötigt, wird über eine Winkeländerung einer mechanischen Spreizvorrichtung in ein elektrisches Signal konvertiert, das zu einem PC übertragen wird. Eine Spracheingabe in den PC reduziert zumindest bei der Florida-Probe das Kontaminationsrisiko bei der Tastatur.

Aus der DE-A-37 12 054 ist ein Messtaster zur Erfassung der Taschentiefe des Zahnfleisches bekannt. Dabei können induktive oder optische Messwertgeber zum Einsatz gelangen, wobei auch Lichtleiter Verwendung finden.

Die Taschentiefe des Zahnfleisches wird nach der US-A-5,100318 mittels Ultraschall gemessen. Gleiches gilt für die US-A-5,755,571, die ein Ultraschallmessgerät zur Taschentiefenmessung vorschlägt. Dabei besteht auch die Möglichkeit, die Lage der Spitze des Messgerätes zu der Tasche mittels eines Sensors zu messen.

Die DE-C-38 36 743 sieht ein kapazitives Messverfahren vor, um die Passgenauigkeit von Kronen- und Brücken-Zahnersatz zu bestimmen.

Um den Abstand zwischen Krone und Spitze einer Zahnwurzel zu ermitteln, wird nach der DE-A-198 54 223 ein Gerät vorgeschlagen, mit dem erste und zweite Messsignale erzeugt werden, aus denen eine Positionsbestimmung erfolgt.

.Aus der US-A-4,673,352 ist eine Vorrichtung zur Messung der Position und Bewegung eines Unterkiefers relativ zum Oberkiefer eines Patienten zu entnehmen. Hierzu werden am Kopf des Patienten ortsfest Empfänger angeordnet, die von einem Messgerät ausgesendete Ultraschallsignale empfangen.

Um die Position eines chirurgischen Instruments zu erfassen, sieht die US-B-6,381,485 einen Bezugs- bzw. Referenzsensor am Kopf eines Patienten vor. Ein Magnetfeldsensor befindet sich sodann am chirurgischen Instrument.

Um einen Kiefer darzustellen ist es nach der EP-A-0 741 994 erforderlich, dass zunächst in einer Mundhöhle eines Patienten eine Vorrichtung zur Lagebestimmung eingebracht wird, die Markierungen bzw. Referenzpunkte aufweist. Mit der Vorrichtung ist ein Sensor verbunden. Ein weiterer Sensor geht vom Unterkiefer des Patienten aus.

Ein System zur Positionserfassung mittels einer an einem Patientenkopf angebrachten Referenzeinheit ist aus der EP-A-0 951 874 bekannt. Hierzu ist am Kopf des Patienten ein Headset befestigt, das eine Referenzeinheit enthält, die über eine Kommunikationsleitung mit einer Positionserfassungseinheit verbunden ist.

Der vorliegenden Erfindung liegt die Aufgabe zu Grunde, ein Verfahren zur Positionsbestimmung eines relativ zu einem Kauorgan eines Patienten zu bewegenden ersten Sensors, mit dem Positionen im Kauorgan oder Bereiche des Kauorgans gemessen werden, insbesondere Positionen an bzw. von Zähnen und/oder Bereiche von Zahntaschen, so weiterzubilden, dass eine unabhängig von dem Patienten vorhandene Referenzierung, insbesondere ein auf einem Ständer zu positionierendes Referenzsystem im Behandlungsraum nicht notwendig ist. Auch soll das Verfahren unempfindlich gegen Metalle im Mundraum sein. Ferner soll im Vergleich zum Stand der Technik die zu verarbeitende Datenmenge reduziert werden, ohne Einbußen der Genauigkeit hinnehmen zu müssen. Die Bewegung des Patienten während des Messens soll zu Messverfälschungen nicht führen.

Bei Verwendung eines intraoralen Scanners als Sensor soll durch Bereitstellung von Grobpositionsdaten die Zusammenführung von Teilmessdaten zu einem einheitlichen 3D Datensatz beschleunigt werden.

Eine weitere Aufgabe ist es, bei Taschentiefenmessungen und/oder einer Karies- /Zahnsteindetektion ein vorzugsweise vollautomatisches Erfassen der Daten zu ermöglichen, insbesondere denn, wenn eine Startposition für die Messungen bekannt ist.

Erfindungsgemäß werden zumindest Teilaspekte des zuvor beschriebenen Problems im Wesentlichen dadurch gelöst, dass als der Positionsbestimmungssensor eine Inertiaplattform verwendet wird und dass die Positionsdaten des Messgeräts unter ergänzender Berücksichtigung zumindest eines in ortsfester Beziehung zum Oberkiefer des Kauorgans angeordneten zweiten Positionsbestimmungssensors bestimmt werden, und dass nach Auslösen eines Startsignals die Positionsdaten der zwei Posnionsbestimmungssensoren synchron ausgewertet werden.

Auch sieht die Erfindung nach einem unabhängigen Lösungsvorschlag vor, dass als der Positionsbestimmungssensor eine Inertialplattform verwendet wird und dass die Bestimmung der Position des Messgerätes mittels des Positionsbestimmungssensors zu einem als Ausgangspunkt gewählten Startsignal erfolgt. Dabei kann das Startsignal räumlich sein, also zu dem Kauorgan eine ortsfeste Beziehung aufweisen.

Ausgehend von einer ersten Positionsmessung, die beispielsweise an einem vorgegebenen Ausgangspunkt als Referenzpunkt erfolgen kann, werden sodann Positionscatenänderungen des Messgerätes mittels weiterer zeitlich versetzter Messungen bestimmt.

Dabei werden die Positionsänderungen des Messgerätes mittels in dem Messgerät integrierter bzw, vorhandener oder zu diesem in bekannter Ortsbeziebung stehender Inertialplattform bestimmt. Es werden drei translatorische und drei rotatorische Freiheitsgrade gemessen, die die Onspositionsänderung des scannenden Sensors zum Kauorgan des Patienten bestimmt. Bevorzugtenweise kann die Positionsänderung unter Berücksichtigung der Daten einer zweiten Inertialplattform erfolgen, die in fester Ortsbeziebung zum Oberkiefer angeordnet ist.

Erfindungsgemäß wird durch Aufintegrieren der Bewegungsänderungen eine neue Relativposition im Vergleich zur ersten Messung berechnet. Die Verkippung im Raum kann ggfs. direkt gemessen werden.

Dabei arbeitet die erfindungsgemäße Lösung nicht unter Einbeziehung von Daten, die mittels bildgebender Verfahren gewonnen wurden, sondern ist sogar in der Lage, selbst dreidimensional bildgebende Daten zu liefern.

Die Verwendung einer Intertialplattform kann zwar den Nachteil zeigen, dass ein zeitliches Driften auftreten kann, wie es von Gyrosystemen in der Luftfahrt bekannt ist. Dies bedarf grundsätzlich eines Nachkalibrierens zu einem festen Koordinatensystem von Zeit zu Zeit. Dieses Driften ist jedoch innerhalb der kurzen Messzeiten, in der die 3D-Daten des Kauorgans aufgenommen werden, so gering, dass dem Grunde nach von einer Korrektur Abstand genommen werden kann.

Für Messungen, bei denen die Bewegung des Patienten als vernachlässigbar im Vergleich zur Messgerät- bzw. Sensorposition angenommen werden kann, reicht die Inertialplattform im Messgerät aus.

Ist die Patientenbewegung nicht vernachlässigbar, ist erfindungsgemäß vorgesehen, dass der weitere Positionsbestimmungssensor (Inertialplattform) am Kopf des Patienten, also in einer ortsfesten Beziehung zum Oberkiefer des Kauorgans, die im Messzeitraum erfolgte Bewegung erfasst. Somit kann die Kopfbewegung des Patienten von der eigentlichen Messgerätebewegung getrennt erfasst werden.

Hierzu kann ein Gestell wie Brillengestell benutzt werden. Auch ein Gesichtsbogen kann zum Befestigen des zweiten Sensors eingesetzt werden. Alternativ kann eine Bissgabel mit einer Inertialplattform am Ober- oder Unterkiefer befestigt werden.

Eine andere Möglichkeit besteht darin, dass ein Beißblock, der die zumindest eine zweite Inertialplattform enthält, zwischen die Zahnreihen gelegt wird und der Patient aufgefordert wird, die Zahnreihen zu schließen.

Dabei ist insbesondere vorgesehen, dass die Positionsdaten als erste Koordinaten des ersten Positionsbestimmungssensors, also ersten Sensors (Inertialplattform) und die Positionsdaten des optionalen zumindest zweiten Positionsbestimmungssensors, also zweiten Sensors (Inertialplattform) als zweite Koordinaten verknüpft werden und dass aus den verknüpften Daten Koordinaten für den ersten Sensor und den zumindest einen zweiten Sensor in einem gemeinsamen Koordinatensystem erzeugt werden, in dem Koordinaten von Positionen oder Bereichen des Kauorgans bestimmt werden.

Bei Messungen am Unterkiefer sollte bevorzugterweise zumindest eine weitere Inertialplattform in fester Ortsbeziehung zum Unterkiefer angebracht werden. Dies kann beispielsweise mit einem Beißblock oder einer am Unterkiefer angebrachten Bissgabel erfolgen. Ungünstig Platzverhältnisse können es im klinischen Einsatz jedoch erfordern, auf eine zum Unterkiefer ortsfeste Positionierung der zumindest einen Inertialplattform zu verzichten. Dies ist dann möglich, wenn die Positionsbestimmung innerhalb der Kauebene ausreichend ist. In diesem Fall kann man, um die Bewegung des Unterkiefers zum Oberkiefer zu berücksichtigen, von der Kenntnis ausgehen, dass der Unterkiefer zu dem Oberkiefer entlang einer bogenförmigen Bewegungsbahn bewegt wird, die vom Kiefergelenk bestimmt wird. Misst man während der Öffnungsbewegung des Unterkiefers einen Messpunkt, beispielsweise zwischen Zahn 31 und 41, weiß man, wie sich der Unterkiefer bewegt. Jeder andere Punkt des Unterkiefers wird sich in einer im mathematischen Sinn ähnlichen Kurve bewegen. Ergänzend kann die typische Mundöffnung berücksichtigt werden, um eine weitere Grobbestimmung vornehmen zu können.

Eine Zahnfleischtaschenposition wird sich in erster Näherung immer auf der gleichen Bogenbahn bewegen.

Durch die mit dem Messgerät (Intraoral-Scanner) fest verbundene Inertialplattform, die Positionsänderungsdaten liefert (ohne oder deutlich genauer mit weiterer ortsfest zum Oberkiefer angeordneter Inertialplattform), kennt man die aktuelle Grobposition des scannenden Sensors zum Kauorgan und die Positionsänderung zu zeitlich früheren Positionen. Damit wird es möglich, das Zusammenfügen von Einzel-3D-Datensätzen zu beschleunigen und in manchen Fällen kritischer Geometrie dies erst möglich zu machen, da die verwendeten Algorithmen gerade bei der groben Bestimmung der Position zweier oder mehrerer Einzeldatensätze zueinander Schwierigkeiten haben. Ist die Grobposition bekannt, kann die folgende Feinjustage der Datensätze zueinander wesentlich einfacher und schneller erfolgen, so dass die Scanndaten mit relativ geringem Rechenaufwand verknüpft werden können.

Erfindungsgemäß steht ein intraorales Scannsystem zur Verfügung, das eine Vorrichtung zur Grobpositionsbestimmung und deren Änderung relativ zum Kauorgan umfasst.

Nach einem eigenerfinderischen Vorschlag kann das intraoral messende Messgerät nicht nur zum Scannen eines Zahnbogens bzw. eines Abschnitts von diesem verwendet werden, um die Position von Zähnen bzw. Bereichen, die mit Zahnrekonstruktionen zu versorgen sind, zu bestimmen, sondern auch zur Bestimmung von Zahntaschentiefen oder Lage und Erstreckung von Karies oder Zahnstein benutzt werden.

Letzteres ist insbesondere dann von Bedeutung, wenn z. B. Zahnfleischtaschen bzw. deren Tiefe gemessen werden sollen, ohne dass ein übliches Erfassen der Daten durch Aufrufen der Messpunkte erfolgt. Vielmehr sind dann, wenn eine Ausgangsposition einer zu messenden Zahntasche bzw. eines Bereichs einer Zahntasche eines Zahns bekannt ist, anschließend automatisch Messungen von Bereichen der Zahntasche desselben Zahns und angrenzende Zähne durchführbar und somit abspeicherbar, da sich die Positionen der sich hieraus ergebenden Ortskoordinaten aus der Stellung des Messgerätes, also des ersten Sensors (Inertialplattform) zu dem in Bezug auf den Oberkiefer ortsfest zumindest einen zweiten Sensor ergibt.

Erfindungsgemäß kann dies voll- bzw. halbautomatisch durchgeführt werden. Wird ein die Taschentiefe messender Abschnitt des Messgerätes in die Tasche geschoben, so kann aufgrund der erfindungsgemäßen Lehre automatisch die Position des Messgerätes ermittelt und abgespeichert werden. Sodann ist es beim halbautomatischen Messen allein erforderlich, dass die Taschentiefen am Messgerät abgelesen werden (z. B. an der Skala einer von dem Messgerät ausgehenden Sonde) und z. B. mittels Spracherkennungssystem an das Patientendokumentationsprogramm im PC übermittelt werden. Die Position des Messgerätes selbst wird automatisch erfasst. Um die automatische Positionserfassung zu ermöglichen, muss eine Ausgangsposition angegeben werden, z. B. Berühren der Gingiva zwischen Zahn 31 und 41 approximal. Ist diese Position bekannt, so wird aufgrund der erfindungsgemäßen Lehre beim Bewegen des Messgerätes die neue Position automatisch relativ zur ersten Position ermittelt, so dass jede Messposition automatisch erfassbar ist. Bei der halbautomatischen Erfassung ist es erwähntermaßen nur erforderlich, dass die Taschentiefe bei jeder Messung z. B. über eine Tastatur eines Rechners eingegeben oder per Spracherkennung übermittelt wird, wohingegen die Position selbst automatisch abgespeichert wird.

Neben der automatischen Positionserfassung des Messortes kann auch der Messvorgang selbst automatisch durchgeführt werden, ohne dass es eines Ablesens von Daten bedarf. Auch hierin ist ein eigenerfinderischer Vorschlag zu sehen. Dies kann auf verschiedene Weise erfolgen, z. B. mit einem Drag-Indikator mit elektronischer Auslesung (z. B. Florida probe), Ultraschalltaschentiefenmessung, optoelektronisch oder mit Impedanzänderungsverfahren.

Messergebnisse, die mit einem entsprechenden Verfahren ermittelt werden, können sodann automatisch mit der Ortsposition in einem Patientenmanagementsystem abgespeichert werden.

Insbesondere erfolgt die Taschentiefenmessung mittels eines optischen Lichtleiters in Form einer oder mehrerer Fasern eines für elektromagnetische Strahlung transparenten Materials z. B. aus Glas, Saphir oder Kunststoff, der in die Tasche eingeführt wird. Dabei kann der Durchmesser des optischen Leiters z. B. im Bereich zwischen 50 µm und 1.000 µm liegen, ohne dass hierdurch eine Einschränkung der erfindungsgemäßen Lehre erfolgt. Von dem Moment an, zu dem der optische Leiter in die Tasche eindringt, ändert sich die von dem Lichtleiter über dessen Stirnfläche gesammelte und einem Empfänger zugeleitete Lichtintensität bzw. spektrale Verteilung aufgrund der optischen Eigenschaften des Zahnfleisches und des Zahnes. Hieraus kann die Eindringtiefe der Spitze des Lichtleiters in die Tasche bestimmt werden, wobei die Messung dann beendet ist, wenn die Stirnfläche des Lichtleiters den Boden der Tasche berührt. Die Taschentiefe berechnet sich dann aus der Differenz zwischen der Position, an der der Eintritt in die Tasche festgestellt wurde und der Position, in der keine weitere Bewegung in z-Richtung (in die Tiefe der Tasche) erfolgt.

Das von dem Lichtleiter erfasste Licht kann Umgebungslicht sein, das z. B. von der Beleuchtung eines Behandlungsstuhles abgegeben wird. Es kann jedoch auch Licht durch den Lichtleiter selbst geleitet und das am Faserende reflektierte Licht mittels einer Photodiode oder eines anderen Lichtdetektors gemessen werden.

Es besteht auch die Möglichkeit, dass das ins Cladding des Lichtleiters rückreflektierte Licht bzw. dessen Veränderung gemessen wird.

Ein weiterer Vorschlag sieht vor, dass in die Tasche zumindest zwei optische Leiter eingeführt werden, wobei über einen oder mehrere der Leiter Licht emittiert und über mindestens einen anderen Leiter Licht empfangen und sodann ausgewertet wird. Hierdurch wird die Nachweisempfindlichkeit des reflektierten Signals erhöht und es kann durch unterschiedliche numerische Aperturen, Durchmesser der Lichtleiter und Abstände zwischen den Lichtleitern zueinander eine Auswahl des Bereiches erfolgen, von wo das in den Lichtleiter reflektierte Licht vorzugsweise empfangen werden soll. Der Abstand zwischen den Lichtleitern beim Einsatz mehrerer Lichtleiter sollte vorzugsweise zwischen dem 0,5- und 3-fachem Durchmesser eines Lichtleiters sein, ohne darauf begrenzt zu sein.

Auch kann eine koaxiale Anordnung eines Lichtleitröhrchens und eines in das Röhrchen lose eingeführten Lichtleiters benutzt werden.

Nach einem anderen Vorschlag ist vorgesehen, dass ein Lichtleiter benutzt wird, dessen Cladding und Coating entfernt sind und der Kern aufgeraut ist. Sodann wird der aufgeraute Lichtleiter von einem Luftmantel oder zumindest einer Beschichtung aus einem Material mit einem geringen Brechungsindex x mit x « 1,3 umgeben. Eine solche Konstruktion stellt sicher, dass die optische Faser in dem aufgerauten Bereich nahezu isotrop abstrahlt.

Alternativ kann an das Ende einer Faser ein Stäbchen aus streuendem Medium angebracht werden, das bei adäquater Wahl des Streukoeffizienten (0,1/mm<µs<100/mm) ebenfalls eine über die Länge des Stäbchens verteilte möglichst homogene Lichtintensität erzeugt.

Sodann besteht die Möglichkeit, die Faser mit Licht einer oder mehrerer Wellenlängen mittels z. B. einer LED, einer Laserdiode oder einer anderen Lichtquelle zu beaufschlagen. Gegebenenfalls besteht auch die Möglichkeit, dass kein Beleuchten der Faser erfolgt, sondern dass diese allein Umgebungslicht aufnimmt. Diesbezügliche Messungen sind möglicherweise mit einem Fehler behaftet.

Wird die Faser mit Licht beaufschlagt, so wird dieses mehr oder weniger isotrop von dem aufgerauten Ende abgestrahlt und entsprechend reflektiertes Licht gesammelt. Sobald der aufgeraute Bereich in die Tasche eindringt, ändert sich die Reflektionsrate von Faser zu Luft gegenüber Faser zum Gewebe und umgekehrt. Dies resultiert in einer Veränderung der Intensität und spektralen Verteilung des reflektierten Lichtes. Der Grad der Veränderung entspricht der Tiefe des Eindringens des Sensors in die Zahntasche. Der Sensor ist zu jeder Zeit aktiv und erfasst unmittelbar das Eindringen in die Tasche. Je tiefer sich der Sensor in der Tasche befindet, umso stärker macht sich eine Signaländerung bemerkbar.

Um die Empfindlichkeit zu erhöhen, können auch zwei entsprechende Fasern, die von Luft oder einer Ummantelung sehr niedrigen Brechungsindexes umgeben sind, verwendet werden, wobei eine Faser als Sensor und die andere Faser als Empfänger in Bezug auf ihre aufgerauten Bereiche dienen. Ein unmittelbarer Lichtübergang vom Sender zum Empfänger wird dadurch verhindert, dass eine Abschirmung zwischen den Sensoren erfolgt. In diesem Fall nimmt der als Empfänger dienende Lichtleiter allein vom Zahn bzw. Gewebe reflektiertes Licht wahr, wobei das reflektierte Licht für jede Wellenlänge unterschiedlich gedämpft und gestreut ist, so dass Rückschlüsse auf die Eigenschaften des Gewebes bzw. des Zahnes erfolgen können, die das Licht reflektieren bzw. von dem Licht durchsetzt sind. Die Unterschiede zwischen den Wellenlängen nehmen in Abhängigkeit von der Eindringtiefe der Faser in das Material zu.

Über die Messung des Spektrums des zurückgestreuten Lichts kann auch festgestellt werden, ob das Gewebe bzw. Zahnfleisch entzündet ist. So ändert sich die Perfusion des Gewebes mit Blut in Abhängigkeit von dem Umfang der Entzündung. Blut hat abweichende optische Eigenschaften zum Gewebe. Werden geeignete Wellenlängen des applizierten Lichtes benutzt, so kann der Umfang der Perfusion ermittelt werden. Zwei Komponenten des Gewebes, und zwar Proteine und Wasser, können als Referenz benutzt werden, wobei für Proteine eine Absorption von Wellenlängen unter 350 nm und für Wasser für Wellenlängen über 1.500 nm bemerkbar ist. Blut hat ein Absorptionsmaximum bei ungefähr 400 nm und eine moderate Absorption im Bereich zwischen 650 nm und 1.000 nm. Wird nun das Verhältnis zwischen reduzierter Blutabsorption im Wellenlängenbereich < 350 nm und Wellenlänge > 1.500 nm zur signifikanten Blutabsorption im Wellenlängenbereich 400 nm bis 1.000 nm gemessen, sind Rückschlüsse auf die Blutperfusion im Gewebe möglich. Diesbezügliche Verfahren können mit jedweder Form zur Bestimmung der Zahntaschentiefe mittels optischer Fasern kombiniert werden.

Die Taschentiefe kann jedoch auch durch Impedanzmessungen ermittelt werden. So kann eine elektrisch leitende Spitze wie beispielsweise eine Parodontalsonde in die Tasche eingeführt werden, wobei gleichzeitig der Gewebewiderstand gemessen wird. Sobald die Sondenspitze die Tasche gefüllt mit Crevicular Flüssigkeit berührt, nimmt der Widerstand ab, z. B. auf einen Wert kleiner als 200 kΩ. In Luft liegt der Widerstand bei größer als 1 MΩ. Die Eindringtiefe der Spitze in die Tasche wird sodann durch die Bewegung des Sensors ermittelt, um auf diese Weise die Taschentiefe zu berechnen. Es kann auch eine Bipolarnadel benutzt werden, die mit einer speichelabweisenden Oberfläche wie Teflon beschichtet ist.

Alternativ besteht die Möglichkeit, einen nichtleitenden Sondengrundkörper zu verwenden, der abschnittsweise leitende Ringabschnitte in unterschiedlichen Abständen von der Sondenspitze aufweist. Während des Eindringens der Sonde in die Tasche wird ein Ring nach dem anderen mit Flüssigkeit bedeckt und die Impedanz zwischen den Ringen ändert sich. Auch kann eine kapazitive Messung durchgeführt werden.

Über einen mittleren oder hohen Widerstandsbelag aufgebraucht als Schicht auf die Sonde kann auch das graduell Eintauchen der Sonde in die Parodontaltasche bemessen werden. Die Eigenschaften eines Potentiometers werden genutzt.

Eine eigenerfinderische Lehre ermöglicht auch die Ortsbestimmung bzw. Flächenerstreckung von Karies bzw. Zahnstein mittels des ersten Positionsbestimmungssensors. Hierbei macht man sich die unterschiedlichen Reflektionseigenschaften von Karies bzw. Zahnstein im Vergleich zum gesunden Zahnbereich zueigen.

Ebenfalls möglich ist die Erfassung der Unterkieferbewegung gegen den Oberkiefer, falls ein Positionsbestimmungssensor (Inertialplattform) am Kopf des Patienten und ein weiterer Positionsbestimmungssensor (Intertialplattform) am Unterkiefer befestigt werden. Insbesondere können hierdurch dynamische Okklusalmessungen durchgeführt werden.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus der nachfolgenden Beschreibung von der Zeichnung zu entnehmenden bevorzugten Ausführungsbeispielen.

Es zeigen:
- Fig. 1: eine Prinzipdarstellung von ersten und zweiten Positionsbestim- mungssensoren, die auf ein Kauorgan ausgerichtet werden,
- Fig. 2: ein Flussdiagramm,
- Fig. 3: eine erste Ausführungsform einer Anordnung zum Messen der Tiefe einer Zahntasche,
- Fig. 4: eine weitere Prinzipdarstellung von Messsensoren,
- Fig. 5: eine dritte Ausführungsform zur Messung einer Zahnfleischtaschentie- fe,
- Fig. 6: eine vierte Ausführungsform zur Messung einer Zahnfleischtaschen- tiefe,
- Fig. 7: eine fünfte Ausführungsform zur Messung einer Zahnfleischtaschen- tiefe,
- Fig. 8 tiefe: eine sechste Ausführungsform zur Messung einer Zahnfleischtaschen- und
- Fig. 9 fe.: eine siebte Ausführungsform zur Messung einer Zahnfleischtaschentie-

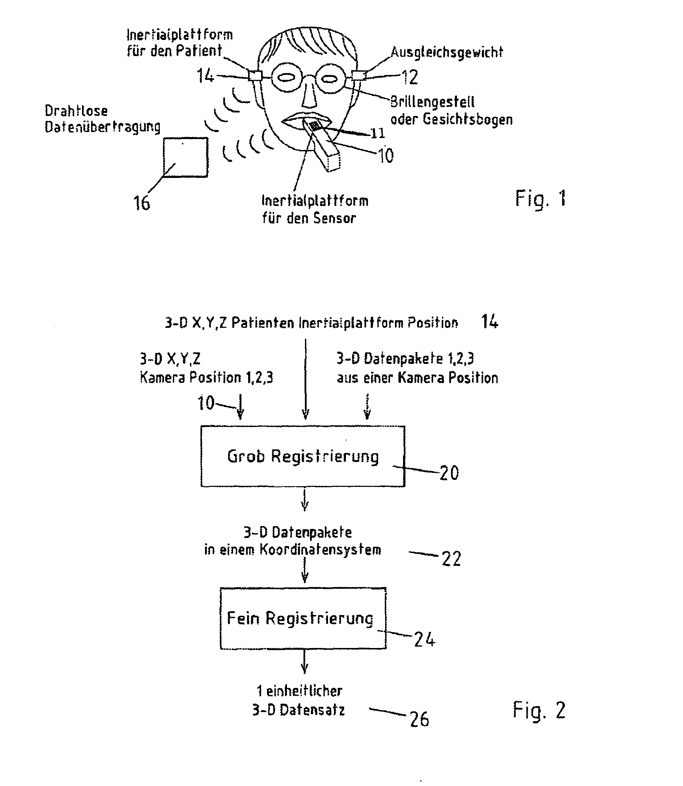

In Fig. 1 ist rein prinzipiell ein als Intraoral-Scanner 10 zu bezeichnendes Messgerät dargestellt, in dem eine Inertialplattform (erster Sensor) integriert ist, durch die die Position des Messgerätes abhängig von dessen Bewegungen in X-, Y- und Z-Richtung sowie Drehung um die jeweiligen Achsen erfassbar ist. Die Inertialplattform kann z. B. mit einem ADIS 16355 aufgebaut sein.

Die Position des Messgerätes 10 und damit des ersten Positionsbestimmungssensors 11 wird zu der Position von zumindest einem zweiten Positionsbestimmungssensor 14 mittels eines Rechners 16 ermittelt, dem die Daten des ersten Positionsbestimmungssensors 11 und des zweiten Positionsbestimmungssensors 14 idealerweise drahtlos übergeben werden. Auch der zweite Sensor 14 beinhaltet eine Inertialplattform. Der zweite Positionsbestimmungssensor 14 kann dabei an dem Bügel einer Brille oder an einem Gesichtsbogen oder in Bissblöcken integriert sein, die zwischen Unter- und Oberkiefer positionierbar sind. Ein Ausgleichsgewicht 12 ist zur symmetrischen Gewichtsverteilung am Brillengestell angebracht.

Somit werden von dem Rechner aus den Daten des ersten Positionsbestimmungssensors 11 und des ortsfest in Bezug auf den Oberkiefer positionierten zweiten Positionsbestimmungssensors 14 die Positionen des Intraoral-Scanners ermittelt und mit den 3D Daten, die an den jeweiligen Positionen gemessen wurden, verknüpft. Damit wird die Grobpositionierung der einzelnen Datensets innerhalb eines gemeinsamen Koordinatensystems beschleunigt (Schritt 20 in Fig. 2). Nach Schritt 20 befinden sich die 3D-Datensets in einem gemeinsamen Koordinatensystem (Schritt 22). Anschließend werden Feinkorrekturen durchgeführt, die die Datensets zueinander in bestmögliche zueinander passende Position bringen (Schritt 24). Der so gewonnene einheitliche 3D-Datensatz 26 kann nun durch weitere bekannte Schritte zur Herstellung von Zahnersatz verwendet werden.

Insbesondere zur digitalen Darstellung von Bereichen des Unter- und/oder Oberkiefer bietet das erfindungsgemäße Verfahren erhebliche Einsparungen in der Rechenzeit und vermeidet Fehldarstellungen; denn mittels der Inertialplattformen oder entsprechend technisch gleichwirkender Ortsbestimmungsmittel werden die Positionen des Intraoral-Scanners in Bezug auf einen zu scannenden Bereich zumindest grob ermittelt, wodurch das Registrieren der Teildatensätze aus unterschiedlichen Aufnahmewinkeln erleichtert wird. Fehlzuordnungen, die bei völlig unbekannter Scannerposition möglich sind, werden hierdurch ausgeschlossen.

Nach dem Ausführungsbeispiel unter Verwendung von zwei Positionsbestimmungssensoren übermittelt der erste Positionsbestimmungssensor Daten (Beschleunigungswerte), mit denen die Ortsveränderungen des Messgerätes in Bezug auf die vorherige Messposition berechnet werden. Der zweite Positionsbestimmungssensor übermittelt Daten, aus denen auf die Ortsveränderungen des Patienten bezügliche dessen Ausgangslage geschlossen werden können.

Selbstverständlich wird die Erfindung nicht verlassen, wenn nur ein Positionsbestimmungssensor eingesetzt wird, dessen Daten Ortsveränderungen zwischen den einzelnen Messorten ermitteln. Dies erfolgt aufgrund der mit der Inertialplattform ermittelten Daten, also der Aufnahme von Beschleunigungswerten, die in Verbindung mit ihrem zeitlichen Verlauf die Berechnung der sich ergebenden Positionsänderungen ermöglichen.

Grundsätzlich werden keine absoluten Positionen in allen 6 Freiheitsgeraden erfasst. Absolute Positionen können jedoch in Bezug auf eine bekannte Startposition bestimmt werden.

Um insbesondere die Tiefe von Zahnfleischtaschen an verschiedenen Orten eines Zahns zu messen, kann das erfindungsgemäße Verfahren, also die Bestimmung der Ortsposition des Messgerätes 10 mit dem ersten Positionsbestimmungssensor allein oder in Verbindung mit einem bzw. dem zweiten Positionsbestimmungssensor 14 benutzt werden, wobei von dem Messgerät 10 ein Sensorelement wie Stift oder optischer Leiter ausgeht, mittels dessen die Taschentiefe gemessen wird. Es besteht jedoch auch die Möglichkeit, mittels Ultraschall die Taschentiefe zu bestimmen. Sender und Empfänger gehen sodann von dem Sensor aus. Ebenso kann mittels einer Impedanzmessung die Taschentiefe gemessen werden.

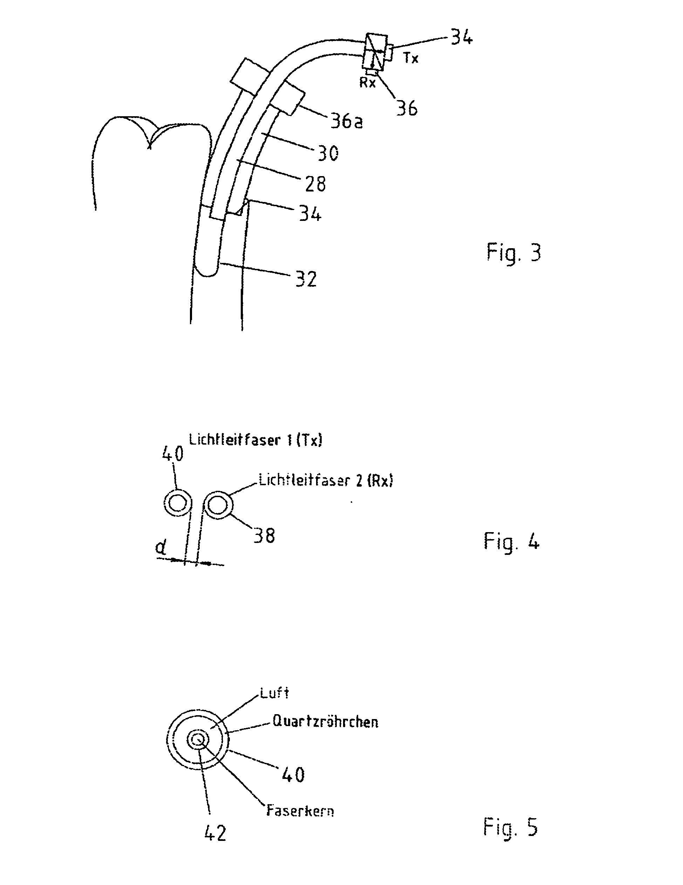

Vorzugsweise kann jedoch opto-elektronisch gemessen werden. Hierzu ist entsprechend der Fig. 3 ein optischer Leiter in Form vorzugsweise einer aus Kunststoff oder Glas bestehenden Lichtleitfaser 28 von einem lichtleitenden Mantel (Coating) 30 umgeben. Der Lichtleiter wird sodann in eine Zahntasche 32 eingeführt. Über den Lichtleiter 28 wird Licht eines Lichtemitters wie mindestens einer Leuchtdiode oder Laserdiode 34 emittiert, um sodann die in der Tasche 32 reflektierte Strahlung über die Faser 28 zurück an den Lichtsensor 36 zu leiten. Ebenso ist über kurze Entfernungen die Rückleitung des reflektierten Lichts über Einkopplung in die Stirnfläche 34 des Coatings 30 zu einem Empfänger möglich. Dies hat den Vorteil der räumlichen Entkopplung von Sender- und Empfängerlichtpfad. Zu dem Zeitpunkt, zu dem die Stirnfläche der Faser die Gingiva berührt bzw. in die Zahnfleischtasche eintaucht, ändert sich das reflektierte Licht in Intensität und im Falle der Verwendung von mehr als einer Wellenlänge auch das Spektrum. Damit ist der Zeitpunkt des Eintritts in die Zahnfleischtasche bekannt. Am Taschenboden stoppt die Bewegung in Taschenrichtung. Die seit dem Eintrittszeitpunkt zurückgelegte Wegstrecke entspricht der Taschentiefe, und kann mittels der bekannten Positionsdaten aus der Inertialplattform ermittelt werden.

Es besteht auch die Möglichkeit, zwei optische Leiter 38, 40 nebeneinander in eine Tasche einzuführen, wobei über einen Leiter, z. B. den optischen Leiter 38, Licht zugeführt wird, um in der Tasche bzw. von dem Gewebe bzw. von dem Zahn, der die Tasche begrenzt, reflektierte Strahlung von dem optischen Leiter bzw. der Faser 40 zu sammeln und einem Empfänger zur Auswertung zuzuleiten. Der Abstand d zwischen den Leitern 38, 40 sollte dabei vorzugsweise 0,5- bis 3-facher Durchmesser eines jeden Leiters 38, 40 sein.

Wie in Fig. 5 dargestellt kann eine koaxiale Ausführungsform genutzt werden. In einem Glas-Saphir oder Quarzröhrchen 40 ist ein Lichtleiter 42 so eingebracht, dass er die Innenfläche des Glasröhrchens nicht berührt. Sobald die Anordnung die Gingiva berührt oder in die Zahnfleischtasche eintaucht, ändert sich das im Material des Röhrchens zurückgeleitete Licht hinsichtlich Intensität und spektraler Verteilung. Damit keine Flüssigkeit in den Luftspalt 40a eindringt, ist die Anordnung mit einem transparenten Fenster aus dem gleichen Material wie das Röhrchen verschlossen.

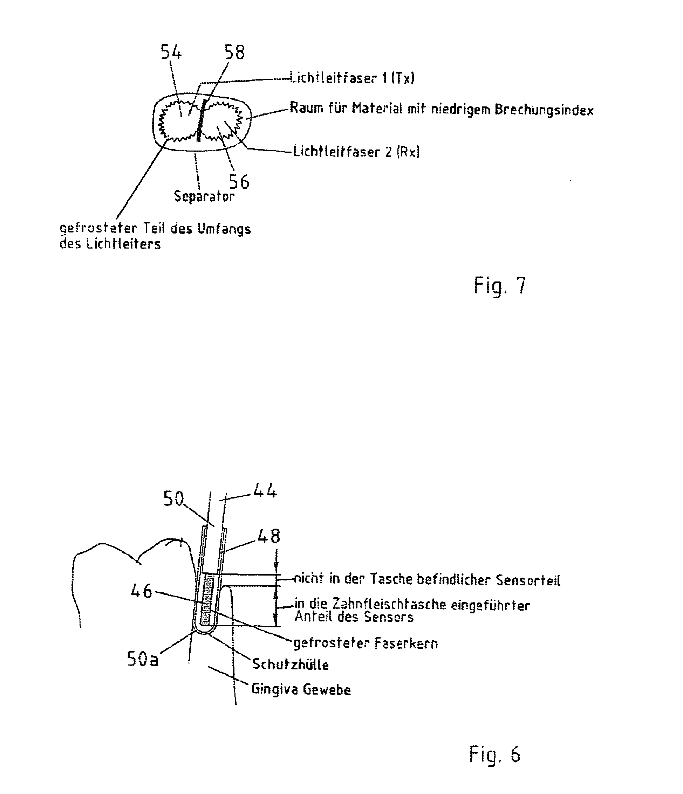

Nach dem Ausführungsbeispiel der Fig. 6 wird ein Lichtleiter 44 benutzt, der in seinem Endbereich 46 aufgeraut ist. Hierzu ist sowohl das Cladding 48 als auch das Coating 50 des Lichtleiters entfernt. Der aufgeraute Endbereich 46 wird sodann in eine Umhüllende 50a und beabstandet zu deren Innenfläche angeordnet, wobei der Zwischenraum mit Luft gefüllt ist. Somit ergibt sich ein Brechungsindexsprung, der eine annähernd gleichmäßige Abstrahlung des Lichts ermöglicht. Anstelle der Umhüllenden 50a mit Luftspalt kann der aufgeraute Abschnitt 46 auch z. B. mit einem Material niedrigen Brechungsindex wie Teflon beschichtet sein.

Der aufgeraute Bereich zeigt den Vorteil, dass Licht annähernd isotrop abgestrahlt und reflektiertes Licht annähernd isotrop gesammelt wird. Sobald der aufgeraute Bereich in die Tasche hinein geschoben wird, ändert sich durch die optischen Eigenschaften des Parodontalgewebes, das vom Licht durchquert wird, die Menge und spektrale Verteilung des rückgestreuten Lichts, in Abhängigkeit von der Eindringtiefe in die Parodontaltasche.

Auf diese Weise kann nicht nur die Taschentiefe, sondern auch mögliche Entzündungen des Zahnfleisches ermittelt werden. Hierzu wird der Lichtleiter mit Strahlung über einen Wellenlängenbereich beaufschlagt, in dem die das Gewebe charakterisierenden Komponenten Protein und Wasser bzw. Blut Strahlung besonders stark absorbieren. Anschließend werden die Intensitäten der charakteristischen Absorptionsbereiche ins Verhältnis gesetzt, um Aussagen über Art und Umfang der Entzündung zu treffen.

Fig. 7 sieht eine Ausführungsform vor, bei der entsprechend der Fig. 6 am Ende vom Coating und Cladding befreiter und aufgerauter Lichtleiter 54 Strahlung in den interessierenden bzw. zu messenden Bereich leitet bzw. abstrahlt und ein ebenso vorbereiteter Lichtleiter 56 aus dem Gebiet reflektierte Strahlung sammelt und einem Empfänger zuführt. Dabei sollten die Lichtleiter 54, 56 in ihren aktiven, also aufgerauten Bereichen optisch voneinander getrennt bzw. abgeschattet sein. Wegen dieser Abschirmung muss das Licht innerhalb des Gewebes einen längeren Weg zurücklegen. Die Anordnung wird somit empfindlicher auf Unterschiede in den optischen Eigenschaften des Gewebes und der Eindringtiefe in die Parodontaltasche.

Auch Impedanzmessungen können zur Bestimmung der Taschentiefe vorgenommen werden. Hierzu kann eine leitende Spitze wie beispielsweise einer Parodontalsonde benutzt werden, deren Impedanz sich in Abhängigkeit von dem Kontakt mit Crevicularflüssigkeit ändert. Berührt die Spitze keine Flüssigkeit, sondern wird in Luft bewegt, so beläuft sich der Widerstand auf mehr als ein 1 MΩ. In dem Moment, wo ein Kontakt mit der Flüssigkeit in der Zahntasche erfolgt, bricht der Widerstand auf Werte kleiner als 200 kΩ zusammen. Diese Widerstandsänderung wird als Indikator für das Eindringen in die Tasche benutzt. Sodann wird die Spitze in die Tasche hinein bis zu deren Boden verstellt und mittels des die Inertialplattform aufweisenden ersten Sensors der Verstellweg bestimmt, um die Tiefe automatisch zu ermitteln. Als Endpunkt dient der Stopp der Bewegung in die Tasche.

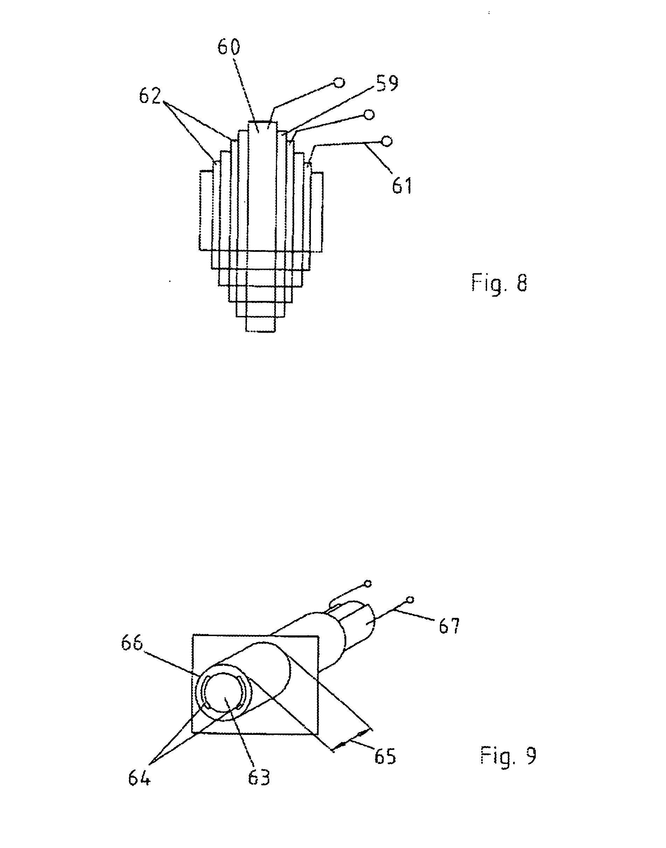

Es besteht auch die Möglichkeit, eine Spitze in die Tasche zu führen, die in parallel verlaufenden und gegeneinander elektrisch isolierten Ebenen Elektroden aufweist, die durch das Gewebe und die in der Tasche befindliche Flüssigkeit elektrisch leitend verbunden werden. Ein entsprechender Aufbau ist in Fig. 8 dargestellt. Um einen leitenden Kern 60 gruppieren sich abwechselnd Isolationsschichten 59 und leitende Schichten 62. So werden leitfähige Ringe erzeugt, die je eine eigene Anschlusselektrode 61 erhalten. Wird die Isolationsschicht zwischen den Elektroden von einer leitfähigen Flüssigkeit benetzt bzw. kommt Gewebe mit zwei benachbarten Elektroden in Berührung, sinkt der Widerstand zwischen den beiden Elektroden deutlich ab. Somit kann die Eindringtiefe der Sonde in eine Flüssigkeit bzw. in die parodontale Tasche schrittweise gemessen werden. Eine sinnvolle Ausführung kann 0,5 mm - 1 mm Eindringtiefenänderung auflösen. Die freien Enden des leitenden Kerns 60 bzw. der leitenden Schichten enden in unterschiedlichen Ebenen, wie sich aus der Zeichnung ergibt, um die Tiefenmessung durchzuführen.

Auch eine kapazitive Messung zur Taschenmessung ist möglich. Hierzu werden auf ein Trägerstäbchen 63 aus einem Material mit niedriger Dielektrizitätskonstante z. B. Teflon, oder Polypropylen gegenüberliegend zwei oder mehrere Elektroden 64 aufgebracht, die mit dem Gewebe, in das die Sonde eintaucht, einen Kondensator bilden. Die Elektroden 64 sind mit einer vorzugsweise hydrophoben Isolierschicht überzogen und mit Verbindungsleitungen 67 mit der Auswerteelektronik verbunden. Abhängig von der Eintauchtiefe 65 ändert sich die Kapazität der Gesamtanordnung.

Lässt man die Isolation 66 in der Ausführung gemäß Fig. 9 weg und führt die Elektroden 64 mit einem mittel- bis hochohmigen Material aus, kann die Eintauchtiefe auch aus der kontinuierlichen Widerstandsänderung der Anordnung bestimmt werden.

Mittels des intraoralen Scannens 10 können nicht nur Positionen im Kauorgan oder Bereiche des Kauorgans wie örtliche Anordnung von Zähnen oder Messpunkte mit gleichzeitiger Tiefenmessung einer Zahntasche automatisch ermittelt werden, sondern es besteht auch die Möglichkeit, Karies bzw. Zahnstein und dessen Erstreckung und Lage zu messen. Hierzu nutzt man die unterschiedlichen Reflexionsspektren von gesundem Zahn im Vergleich zu Bereichen, die von Karies befallen sind bzw. Zahnstein aufweisen, die bei der Ortsbestimmung des ersten Sensors mit ausgewertet werden können.

## Patentansprüche

1. Verfahren zur Positionsbestimmung eines relativ zu einem Kauorgan eines Patienten zu bewegenden intraoral messenden Messgeräts, mit dem Positionen im Kauorgan oder Bereiche des Kauorgans gemessen werden, insbesondere zur Taschentiefenmessung von Zahnfleisch und/oder Karies- bzw. Zahnsteindetektion, wobei die Position des Messgerätes mittels eines in ortsfester Beziehung zu dem Messgerät stehenden Positionsbestimmungssensors gemessen wird,
**dadurch gekennzeichnet,**
**dass** als der Positionsbestimmungssensor eine Inertialplattform verwendet wird und dass Positionsdaten des Messgeräts unter ergänzender Berücksichtigung zumindest einer in ortsfester Beziehung zum Oberkiefer des Kauorgans angeordneten zweiten Inertialplattform bestimmt werden, und dass nach Auslösen eines Startsignals die Positionsdaten der zwei Inertialplattformen synchron ausgewertet werden.

2. Verfahren zur Positionsbestimmung eines relativ zu einem Kauorgan eines Patienten zu bewegenden intraoral messenden Messgeräts, mit dem Positionen im Kauorgan oder Bereiche des Kauorgans gemessen werden, insbesondere zur Taschentiefenmessung von Zahnfleisch und/oder Karies- bzw. Zahnsteindetektion, wobei die Position des Messgerätes mittels eines in ortsfester Beziehung zu dem Messgerät stehenden Positionsbestimmungssensors gemessen wird,
**dadurch gekennzeichnet,**
**dass** als der Positionsbestimmungssensor eine Inertialplattform verwendet wird und dass die Bestimmung der Position des Messgerätes mittels der Inertialplattform zu einem als Ausgangspunkt gewählten Startsignal erfolgt, das in ortsfester Beziehung zu dem Kauorgan steht.

3. Verfahren nach Anspruch 1,
**dadurch gekennzeichnet,**
**dass** die Positionsdaten der ersten Inertialplattform als erste Koordinaten und die Positionsdaten der zumindest einen zweiten Inertialplattform als zweite Koordinaten verknüpft werden und dass aus den verknüpften Daten Koordinaten für die erste Inertialplattform und die zumindest eine zweite Inertialplattform in einem gemeinsamen Koordinatensystem erzeugt werden, in dem Koordinaten von Positionen oder Bereichen des Kauorgans bestimmt werden.

4. Verfahren nach Anspruch 1 ,
**dadurch gekennzeichnet,**
**dass** die zumindest eine zweite Inertialplattform an einem Gestell wie Brillengestell oder an einem Gesichtsbügel oder in einen Bissblock befestigt wird, der zwischen Unter- und Oberkiefer angeordnet wird.

5. Verfahren nach zumindest einer der vorhergehenden Ansprüche,
**dadurch gekennzeichnet,**
**dass** zur Bestimmung von Ortskoordinaten von Bereichen des Unterkiefers dessen Bewegung entlang eines vom Oberkiefer ausgehenden Bogens ergänzend berücksichtigt wird.

6. Verfahren nach Ansprüch 2,
**dadurch gekennzeichnet,**
**dass** zur Bestimmung von Ortskoordinaten von Bereichen des Unterkiefers und/oder Oberkiefers eine weitere Inertialplattform ortsfest zu dem Unter- bzw. Oberkiefer insbesondere am Kopf des Patienten angeordnet wird.

7. Verfahren nach zumindest einer der vorhergehenden Ansprüche,
**dadurch gekennzeichnet,**
**dass** mit dem Messgerät vorzugsweise mittels Ultraschall, Drag-Indikator, optoelektronisch, induktiv, kapazitiv oder durch Widerstandsmessung die Taschentiefe eines Zahns gemessen wird, wobei insbesondere das Messgerät ein stiftförmiges Element aufweist, mit dem Tiefe und/oder Zustand der Zahntasche durch Ortsveränderung des Messgerätes elektrisch und/oder optoelektronisch gemessen wird.

8. Verfahren nach zumindest Anspruch 7,
**dadurch gekennzeichnet,**
**dass** mit dem Messgerät die Taschentiefe in Abhängigkeit von der Position der ersten Inertialplattform zu der zweiten Inertialplattform gemessen wird.

9. Verfahren nach zumindest Anspruch 7,
**dadurch gekennzeichnet,**
**dass** ein optischer Lichtleiter in die Tasche eingeführt wird und dass die Tiefe der Tasche in Abhängigkeit von über den Lichtleiter einem Empfänger zugeführtem Licht bestimmt wird, wobei insbesondere Änderungen von Intensität und/oder spektrale Verteilung des über den optischen Lichtleiter dem Empfänger zugeleiteten Lichts zur Bestimmung der Taschentiefe ausgewertet werden.

10. Verfahren nach zumindest Anspruch 7,
**dadurch gekennzeichnet,**
**dass** ein optischer Leiter zur Beleuchtung und Rückleitung des rückgestreuten Lichts zur Messung der Taschentiefe verwendet wird.

11. Verfahren nach zumindest Anspruch 9 oder 10,
**dadurch gekennzeichnet,**
**dass** vom optischen Leiter empfangenes Umgebungslicht zur Messung der Taschentiefe ausgewertet wird, wobei vorzugsweise der optische Leiter von einem lichtleitenden Mantel umgeben ist, über dessen Stirnfläche eingekoppeltes Licht zur Messung der Taschentiefe ausgewertet wird.

12. Verfahren nach zumindest Anspruch 7,
**dadurch gekennzeichnet,**
**dass** in die Tasche zumindest zwei optische Leiter eingeführt werden, wobei über einen der Leiter Licht zugeführt und über den anderen Leiter empfangenes Licht ausgewertet wird.

13. Verfahren nach zumindest einem der Ansprüche 9 bis 12,
**dadurch gekennzeichnet,**
**dass** als optischer Leiter eine Faser oder ein Faserbündel verwendet wird, wobei der optische Leiter einen Durchmesser D mit 50 µm ≤ D ≤ 1000 µm aufweist und dass bei der Verwendung von zwei optischen Leitern diese einen lichten Abstand einnehmen, der 0,5 D ≤ d ≤ 3 D mit D gleich Durchmesser des optischen Leiters ist.

14. Verfahren nach zumindest einem der Ansprüche 9 bis 13,
**dadurch gekennzeichnet,**
**dass** ein optischer Leiter in einer koaxialen Anordnung innerhalb eines transparenten Röhrchens z. B. aus Saphir positioniert wird, ohne dieses zu berühren.

15. Verfahren nach zumindest einem der Ansprüche 9 bis 14,
**dadurch gekennzeichnet,**
**dass** als optischer Leiter eine in ihrem freien Ende von Cladding und Coating befreite aufgeraute lichtleitende Faser verwendet wird, dass der optische Leiter mit Licht vorzugsweise ausgewählter Wellenlänge oder ausgewählten Wellenlängenbereichs, insbesondere im Bereich von 350 nm ≤ λ ≤ 10.000 nm, vorzugsweise 400 nm ≤ λ ≤ 2000 nm beaufschlagt wird, und dass das über das aufgeraute Ende in den optischen Leiter zurückreflektierte Licht zur Bestimmung der Taschentiefe ausgewertet wird.

16. Verfahren nach zumindest einem der Ansprüche 9 bis 15,
**dadurch gekennzeichnet,**
**dass** zwei bereichsweise aufgeraute optische Leiter in die Tasche eingeführt werden, dass über einen Leiter Licht in die Tasche und über den anderen Leiter in der Tasche reflektiertes Licht zu einem Empfänger geleitet wird, wobei insbesondere die optischen Leiter in ihren aufgerauten Bereichen gegeneinander abgeschirmt werden.

17. Verfahren nach zumindest einem der Ansprüche 9 bis 16,
**dadurch gekennzeichnet,**
**dass** das über den optischen Leiter dem Empfänger zugefuhrte Licht in Bezug auf das Absorptionsverhalten der Tasche ausgewertet wird, wobei insbesondere das Absorptionsverhältnis von Strahlung im Wellenlängenbereich λ₁, λ₂ mit λ₁ ≤ 350 nm und λ₂ > 1500 nm zur Strahlung im Wellenlängenbereich Δλ mit 400 nm ≤ Δ λ ≤ 1000 nm bestimmt wird.

18. Verfahren nach zumindest Anspruch 7,
**dadurch gekennzeichnet,**
**dass** Widerstandsänderung eines in die Tasche eingeführten leitenden Messsensors in Abhängigkeit von dessen Eindringtiefe in die Tasche gemessen wird.

19. Verfahren nach zumindest Anspruch 7,
**dadurch gekennzeichnet,**
**dass** ein mit einer hydrophoben Beschichtung überzogener Messsensor aus elektrisch isolierendem Material mit zueinander beabstandeten Elektrodenabschnitten in Ebenen gleichen Querschnitts des Messsensors in die Tasche eingeführt wird und dass Impedanzänderung zwischen den Elektrodenabschnitten zur Bestimmung der Taschentiefe gemessen wird.

20. Verfahren nach zumindest Anspruch 7,
**dadurch gekennzeichnet,**
**dass** ein mit einer hydrophoben Beschichtung überzogener Messsensor aus elektrisch isolierendem Material mit zueinander beabstandeten in Sensorlängsrichtung streifenförmigen Elektrodenabschnitten des Messsensors in die Tasche eingeführt wird und dass Impedanzänderung zwischen den Elektrodenabschnitten zur Bestimmung der Taschentiefe gemessen wird.

21. Verfahren nach zumindest Anspruch 7,
**dadurch gekennzeichnet,**
**dass** spektrale Verteilung der empfangenen Strahlung von dem Messgerät zur Ortsbestimmung und/oder Erstreckung von Karies und/oder Zahnstein ausgewertet wird.

## Claims

1. Method for determining the position of an intraorally measuring measuring device to be moved relative to a patient's masticatory organ, which is used to measure positions in the masticatory organ or regions of the masticatory organ, in particular to measure the depth of gingival pockets and/or for detection of caries, respectively plaque, whereby the position of the measuring device is measured by means of a position finding sensor in a stationary position relative to the measuring device,
**characterized in**
**that** as the position finding sensor is used an inertial platform and that positional data of the measuring device is determined by taking into additional account at least one second inertial platform arranged in a stationary position relative to the maxilla of the masticatory organ, and that after triggering of a starting signal the positional data of the two inertial platforms are evaluated synchronously.

2. Method for determining the position of an intraorally measuring measuring device to be moved relative to a patient's masticatory organ, which is used to measure positions in the masticatory organ or regions of the masticatory organ, in particular to measure the depth of gingival pockets and/or for detection of caries, respectively plaque, whereby the position of the measuring device is measured by means of a position finding sensor in a stationary position relative to the measuring device,
**characterized in**
**that** as the position finding sensor is used an inertial platform and that the determination of the position of the measuring device is made by means of the inertial platform relative to a starting signal chosen as a starting point, said starting signal being in a stationary relation to the masticatory organ.

3. Method according to claim 1,
**characterized in**
**that** the positional data of the first inertial platform as first coordinates and the positional data of the at least one second inertial platform as second coordinates are linked, and that from the linked data are generated coordinates in a common coordinate system for the first inertial platform and the at least one second inertial platform, in which coordinates of positions or regions of the masticatory organ are determined.

4. Method according to claim 1,
**characterized in**
**that** the at least one second inertial platform is attached to a frame such as a spectacles frame or to a facebow, or in a bite block that is arranged between mandible and maxilla.

5. Method according to at least one of the preceding claims,
**characterized in**
**that** for determining position coordinates of regions of the mandible, the movement of the mandible along an arch extending from the maxilla is additionally taken into account.

6. Method according to claim 2,
**characterized in**
**that** for determining position coordinates of regions of the mandible and/or maxilla one additional inertial platform is arranged especially at the patient's head in a position that is stationary relative to the mandible or maxilla.

7. Method according to at least one of the preceding claims,
**characterized in**
**that** with the measuring device, preferably by means of ultrasound, drag indicator, opto-electronic, inductive, capacitive, or resistance measurements, the pocket depth of a tooth is determined, with especially the measuring device comprising a pin-shaped element by means of which depth and/or condition of the gingival pocket is measured electrically and/or opto-electronically by varying the position of the measuring device.

8. Method according to at least claim 7,
**characterized in**
**that** the measuring device is used to measure the pocket depth in dependence on the position of the first inertial platform relative to the second inertial platform.

9. Method according to at least claim 7,
**characterized in**
**that** an optical light guide is inserted into the pocket and that the depth of the pocket is determined in dependence on light fed via the light guide to a receiver, whereby especially changes in intensity and/or spectral distribution of the light directed to the receiver via the optical light guide are analyzed to determine the pocket depth.

10. Method according to at least claim 7,
**characterized in**
**that** for measuring the pocket depth an optical guide is used for illumination and for returning the backscattered light.

11. Method according to at least claims 9 or 10,
**characterized in**
**that** ambient light received by the optical guide is analyzed to measure the pocket depth, whereby preferably the optical guide is surrounded by a light-conducting cladding via whose front face light coupled-in is analyzed to measure the pocket depth.

12. Method according to at least claim 7,
**characterized in**
**that** at least two optical guides are inserted into the pocket, whereby light is supplied via one of the guides and light received via the other guide is evaluated.

13. Method according to at least one of claims 9 to 12,
**characterized in**
**that** used as optical guide is a fiber or fiber bundle, with the optical guide having a diameter D with 50 µm ≤ D ≤ 1000µm and that when using two optical guides, these are positioned with a clearance, which is 0.5 D ≤ d ≤ 3 D where D equals the diameter of the optical guide.

14. Method according to at least one of claims 9 to 13,
**characterized in**
**that** an optical guide is positioned in a coaxial arrangement inside of a transparent small tube, of e.g. sapphire, without coming into contact with the small tube.

15. Method according to at least one of claims 9 to 14,
**characterized in**
**that** as optical guide is used a light-conducting fiber that at its free end has been freed of cladding and coating and has been roughened, that the optical guide is impinged with light of a preferably selected wavelength or selected wavelength region, in particular in the region of 350 µm ≤ λ ≤ 10,000 nm, preferably 400 nm ≤ λ ≤ 2000 nm, and that the light reflected back into the optical guide via the roughened end is analyzed to determine the pocket depth.

16. Method according to at least one of claims 9 to 15,
**characterized in**
**that** two regionally roughened optical guides are inserted into the pocket, that light is directed into the pocket via one guide and light reflected in the pocket is directed to a receiver via the other guide, whereby especially the optical guides are screened relative to each other in their roughened regions.

17. Method according to at least one of claims 9 to 16,
**characterized in**
**that** the light fed to the receiver via the optical guide is evaluated with regard to the absorption characteristics of the pocket, whereby determined is especially the ratio of absorption of radiation in the wavelength region λ₁, λ₂, with λ₁ ≤ 350 nm and λ₂ >1500 nm relative to the radiation in the wavelength region Δλ with 400 nm ≤ Δλ ≤ 1000 nm.

18. Method according to at least claim 7,
**characterized in**
**that** change in resistance of a conducting measuring sensor introduced into the pocket is measured in dependence on its penetration depth into the pocket.

19. Method according to at least claim 7,
**characterized in**
**that** a measuring sensor, coated with a hydrophobic coating and consisting of an electrically insulating material and having spaced-apart electrode sections that are arranged in planes of equal cross-section of the measuring sensor, is inserted into the pocket, and that a change in impedance between the electrode sections is measured to determine the pocket depth.

20. Method according to at least claim 7,
**characterized in**
**that** a measuring sensor, coated with a hydrophobic coating and consisting of an electrically insulating material and having spaced-apart band-like electrode sections extending along the sensor's longitudinal direction, is inserted into the pocket, and that a change in impedance between the electrode sections is measured to determine the pocket depth.

21. Method according to at least claim 7,
**characterized in**
**that** the spectral distribution of the received radiation is evaluated by the measuring device to determine the location and/or extent of caries and/or dental calculus.

## Revendications

1. Procédé destiné à déterminer la position d'un appareil de mesure mesurant intraoralement et devant être déplacé relativement à un organe de mastication d'un patient, avec lequel sont mesurées des positions dans l'organe de mastication ou dans des zones de l'organe de mastication, en particulier pour mesurer la profondeur de poches parodontales et/ou détecter des caries ou du tartre dentaire, sachant que la position de l'appareil de mesure est mesurée au moyen d'un capteur de position fixe par rapport à l'appareil de mesure,
**caractérisé en ce**
**qu'**une plateforme inertielle est utilisée en tant que capteur de position et que les données de position de l'appareil de mesure sont déterminées avec prise en considération complémentaire d'au moins une deuxième plateforme inertielle fixe par rapport à la mâchoire supérieure de l'organe de mastication, et qu'après le déclenchement d'un signal de lancement, les données de position des deux plateformes inertielles sont analysées de manière synchronisée.

2. Procédé destiné à déterminer la position d'un appareil de mesure mesurant intraoralement et devant être déplacé relativement à un organe de mastication d'un patient, avec lequel sont mesurées des positions dans l'organe de mastication ou dans des zones de l'organe de mastication, en particulier pour mesurer la profondeur de poches parodontales et/ou détecter des caries ou du tartre dentaire, sachant que la position de l'appareil de mesure est mesurée au moyen d'un capteur de position fixe par rapport à l'appareil de mesure,
**caractérisé en ce**
**qu'**une plateforme inertielle est utilisée en tant que capteur de position et que la détermination de la position de l'appareil de mesure est effectuée au moyen de la plateforme inertielle à partir d'un signal de lancement choisi comme point de départ fixe par rapport à l'organe de mastication.

3. Procédé selon la revendication 1,
**caractérisé en ce**
**que** les données de position de la première plateforme inertielle sont enchaînées en tant que premières coordonnées et les données de position de l'au moins une deuxième plateforme inertielle sont enchaînées en tant que deuxièmes coordonnées et qu'à partir de ces données enchaînées, des coordonnées pour la première plateforme inertielle et l'au moins une deuxième plateforme inertielle sont générées dans un système de coordonnées commun à l'intérieur duquel sont déterminées des coordonnées de positions ou de zones de l'organe de mastication.

4. Procédé selon la revendication 1,
**caractérisé en ce**
**que** l'au moins une deuxième plateforme inertielle est fixée sur une monture telle qu'une monture de lunettes, ou sur un arc facial, ou dans un bloc de mordu placé entre la mâchoire inférieure et la mâchoire supérieure.

5. Procédé selon au moins une des revendications précédentes, **caractérisé en ce**
**que** pour déterminer des coordonnées locales de zones de la mâchoire inférieure, le mouvement de cette dernière le long d'un arc partant de la mâchoire supérieure est pris en compte de manière complémentaire.

6. Procédé selon la revendication 2,
**caractérisé en ce**
**que** pour déterminer des coordonnées locales de zones de la mâchoire inférieure et/ou de la mâchoire supérieure, une plateforme inertielle additionnelle est disposée de manière fixe par rapport à la mâchoire inférieure ou à la mâchoire supérieure, en particulier sur la tête du patient.

7. Procédé selon au moins une des revendications précédentes,
**caractérisé en ce**
**qu'**avec l'appareil de mesure, la profondeur de la poche d'une dent est de préférence mesurée au moyen d'ultrason, d'aiguille entraînée, de mesure optoélectronique, inductive, capacitive ou de résistance, sachant que l'appareil de mesure présente en particulier un élément en forme de tige avec lequel la profondeur et/ou l'état de la poche parodontale est/sont mesuré(e)(s) électriquement et/ou optoélectroniquement par déplacement de l'appareil de mesure.

8. Procédé selon au moins la revendication 7,
**caractérisé en ce**
**qu'**avec l'appareil de mesure est mesurée la profondeur de poche en fonction de la position de la première plateforme inertielle par rapport à la deuxième plateforme inertielle.

9. Procédé selon au moins la revendication 7,
**caractérisé en ce**
**qu'**un conducteur optique est introduit dans la poche et que la profondeur de la poche est déterminée en fonction de la lumière acheminée vers un récepteur par le conducteur optique, sachant qu'en particulier des variations de l'intensité et/ou de la répartition spectrale de la lumière acheminée vers le récepteur par le conducteur optique sont analysées pour déterminer la profondeur de la poche.

10. Procédé selon au moins la revendication 7,
**caractérisé en ce**
**qu'**un conducteur optique destiné à éclairer et guider en retour la lumière rétrodiffusée est utilisé pour mesurer la profondeur de poche.

11. Procédé selon au moins la revendication 9 ou 10,
**caractérisé en ce**
**que** la lumière ambiante reçue par le conducteur optique est analysée pour mesurer la profondeur de poche, sachant que de préférence le conducteur optique est entouré d'une enveloppe guidant la lumière et qui analyse la lumière injectée par sa face frontale pour mesurer la profondeur de poche.

12. Procédé selon au moins la revendication 7,
**caractérisé en ce**
**qu'**au moins deux conducteurs optiques sont introduits dans la poche, sachant que de la lumière est acheminée par l'un des conducteurs et que la lumière reçue par l'autre conducteur est analysée.

13. Procédé selon au moins une des revendications 9 à 12 précédentes,
**caractérisé en ce**
**qu'**en tant que conducteur optique est utilisé(e) une fibre ou un faisceau de fibres, sachant que le conducteur optique présente un diamètre D tel que 50 µm ≤ D ≤ 1000 µm, et qu'en cas d'utilisation de deux conducteurs optiques, ces derniers présentent entre eux un écartement tel que 0,5 D ≤ d ≤ 3 D, où D est égal au diamètre du conducteur optique.

14. Procédé selon au moins une des revendications 9 à 13 précédentes,
**caractérisé en ce**
**qu'**un conducteur optique est positionné de manière coaxiale à l'intérieur d'un tube transparent, p. ex. en saphir, sans le toucher.

15. Procédé selon au moins une des revendications 9 à 14 précédentes,
**caractérisé en ce**
**qu'**en tant que conducteur optique est utilisée une fibre guidant la lumière, rendue rugueuse, libérée de gaine et de revêtement à ses extrémités libres, que le conducteur optique est alimenté en lumière de préférence d'une longueur d'onde sélectionnée ou d'une plage de longueurs d'ondes sélectionnée, en particulier dans la plage de 350 nm ≤ λ ≤ 10 000 nm, de préférence de 400 nm ≤ λ ≤ 2000 nm, et que la lumière rétroréfléchie dans le conducteur par l'extrémité rugueuse est analysée pour déterminer la profondeur de poche.

16. Procédé selon au moins une des revendications 9 à 15 précédentes,
**caractérisé en ce**
**que** deux conducteurs optiques partiellement rendus rugueux sont introduits dans la poche, que de la lumière est guidée par un conducteur dans la poche et que la lumière réfléchie dans la poche est guidée par l'autre conducteur vers un récepteur, sachant qu'en particulier les zones rugueuses des conducteurs optiques sont obscurcies l'une par rapport à l'autre.

17. Procédé selon au moins une des revendications 9 à 16 précédentes,
**caractérisé en ce**
**que** la lumière guidée vers le récepteur par le conducteur optique est analysée en ce qui concerne le comportement d'absorption de la poche, sachant qu'est en particulier déterminé le rapport d'absorption entre le rayonnement dans la plage de longueurs d'ondes λ₁, λ₂, où λ₁ ≤ 350 nm et λ₂ > 1500 nm, et le rayonnement dans la plage de longueurs d'ondes Δλ, où 400 nm ≤ Δλ ≤ 1000 nm.

18. Procédé selon au moins la revendication 7,
**caractérisé en ce**
**que** la variation de la résistance d'un capteur de mesure conducteur introduit dans la poche est mesurée en fonction de la profondeur de pénétration dudit capteur dans la poche.

19. Procédé selon au moins la revendication 7,
**caractérisé en ce**
**qu'**est introduit dans la poche un capteur de mesure en matériau électro-isolant et revêtu d'une couche hydrophobe, avec des segments d'électrodes distants les uns des autres dans des plans de même section du capteur de mesure, et que la variation d'impédance entre les segments d'électrodes est mesurée pour déterminer la profondeur de la poche.

20. Procédé selon au moins la revendication 7,
**caractérisé en ce**
**qu'**est introduit dans la poche un capteur de mesure en matériau électro-isolant et revêtu d'une couche hydrophobe, avec des segments d'électrodes du capteur de mesure distants les uns des autres et en forme de lame dans le sens longitudinal du capteur, et que la variation d'impédance entre les segments d'électrodes est mesurée pour déterminer la profondeur de la poche.

21. Procédé selon au moins la revendication 7,
**caractérisé en ce**
**que** la répartition spectrale du rayonnement reçu de l'appareil de mesure est analysée pour déterminer la position des caries et/ou du tartre dentaire et/ou déterminer leur étendue.
